# EUROPÄISCHE PATENTSCHRIFT

(11) **EP 2 383 005 B1**
(45) Veröffentlichungstag und Bekanntmachung des Hinweises auf die Patenterteilung: **24.02.2016**
(21) Anmeldenummer: 11001648.2
(22) Anmeldetag: 01.03.2011
(51) Int. Cl.: A61M 5/00

(54) **Vorfüllspritze**
Filling injection device
Seringue de préremplissage

(30) Priorität: 10.03.2010 DE 102010010967
(43) Veröffentlichungstag der Anmeldung: 02.11.2011
(73) Patentinhaber: Balda Solutions GmbH, 32549 Bad Oeynhausen (DE)
(72) Erfinder: Schliemann, Eric, 78256 Steisslingen (DE); Fuchs, Karl-Heinz, 78315 Radolfzell (DE)
(74) Vertreter: Isarpatent

(56) Entgegenhaltungen:
- FR-A1- 2 356 432
- US-A- 2 607 341
- US-A- 2 725 057
- US-A- 3 107 785

## Beschreibung

### Technisches Gebiet

Die vorliegende Erfindung betrifft eine Vorfüllspritze nach den Merkmalen des Oberbegriffs des Anspruchs 1 sowie der nebengeordneten Verwendungsansprüche zur Nutzung dieser.

### Stand der Technik

Als Stand der Technik wird auf die US 2 607 341 A, sowie auf die US 2 725 057 A hingewiesen. Beide Schriften offenbaren eine Spritzenvorrichtung, wobei der Einsatz eines Deckels als Stössel vorgesehen ist.

Aus dem Stand der Technik sind vielfältige so genannte Vorfüllspritzen bekannt. Nachteilig hierbei ist allerdings, dass aufgrund des im Spritzenkörper befindlichen Mediums der zur Spritze dazugehörige Kolben nicht im Spritzenkörper gelagert werden kann, sondern in der Regel in der Weise transportiert wird, dass der Kolben aus dem Spritzenkörper herausgezogen ist und der Nutzer dann den Kolben durch Niederdrücken betätigt und das Medium aus dem Spritzenkörper drückt. Durch den ausgezogenen Kolben bedarf es hier aber eines beträchtlichen Materialaufwandes für die Verpackung und Platzaufwandes für den Transport.

### Aufgabe

Aufgabe der vorliegenden Erfindung ist es, Nachteile des Standes der Technik abzustellen und oben genannte Vorfüllspritzen zu verbessern und dabei möglichst eine kompakte Ausgestaltung zu erhalten.

### Lösung der Aufgabe

Zur Lösung der Aufgabe führen die Merkmale des kennzeichnenden Teils des Anspruchs 1, sowie der nebengeordneten Verwendungsansprüche.

Eine Vorfüllspritze wird vorgefüllt mit einem Fluid an den Nutzer geliefert. Das Fluid soll zwar in erster Linie medizinischen Belangen genügen, doch ist es ebenfalls möglich kosmetischen Belangen mit dem Fluid Genüge zu tun.

Ein Transportzustand ist vorzugsweise ein Zustand in dem üblicherweise die erfindungsgemäße Vorfüllspritze vom Hersteller verpackt und an den Nutzer versandt wird. Ein Arbeitszustand ist vorzugsweise ein Zustand in dem die Vorfüllspritze durch einen Nutzer betätigt wird.

Die Vorfüllspritze besteht aus einer Aufnahmehülse mit einer Düse und einem Kolben. Der Kolben soll bevorzugt in der Weise ausgebildet sein, dass er in einem Transportzustand über das düsen-seitige Ende der Aufnahmehülse gesteckt ist. Vorteilhaft hierbei ist, dass durch diese Steckverbindung ein Platzersparnis erreicht wird, da herkömmliche Vorfüllspritzen mit einem ausgezogenen Kolben an den Nutzer versandt werden muss, um das Fluid mit senden zu können.

In einem bevorzugten Ausführungsbeispiel können Teile des Kolbens durch die Düse auch in das Innere der Aufnahmehülse ragen. Ebenso können die gleichen Teile dazu dienen die Düse der Aufnahmehülse während des Transpostzustands zu verschließen.

Außerdem soll der Kolben in der Weise ausgebildet sein, dass er in einem Arbeitszustand zumindest teilweise in die Aufnahmehülse einschiebbar ist. Arbeitszustand bedeutet hierbei das Ausbringen des Fluids aus der Vorfüllspritze. Dies kann auf einmal oder in mehrere Stufen geschehen. Vorteilhaft hierbei ist, dass der Kolben während des Transpostzustand zunächst platzsparend am Düsen-seitigen Ende aufgeschoben ist und der Nutzer zum Ausbringen des Fluids den Kolben im Arbeitszustand zum Ausbringen des Fluids am dem Düsen-seitigen Ende abgewandten Bereich in die Aufnahmehülse schieben kann.

Der Kolben kann hierbei auch die Düse während des Transpostzustands verschließen und während des Arbeitszustands öffnen. Es ist auch möglich, dass der Kolben mehrmals abwechselnd entweder am Düsen-seitigen Ende oder dem dem Düsen-seitigen Ende abgewandten Ende entweder über die Aufnahmehülse oder in die Aufnahmehülse auf- oder eingeschoben werden kann.

Der Umfang des Kolbens soll in einem bevorzugten Ausführungsbeispiel veränderbar sein. Während des Transportzustands soll der Kolben einen größeren Umfang aufweisen als die Aufnahmehülse und während des Arbeitszustands soll der Kolben einen geringeren Umfang als der Innenumfang der Aufnahmehülse aufweisen und geeignet sein ganz oder teilweise in die Aufnahmehülse zu verfahren. Dies ermöglicht den Vorteil der Platzersparnis.

Ein bevorzugtes Ausführungsbeispiel weist eine Dichtung im Inneren der Aufnahmehülse auf. Diese Dichtung verhindert zum einen vorteilhaft, dass das Fluid auf der dem Düsen-seitigen Ende abgewandtem Seite ausläuft. Außerdem wirkt die Dichtung mit dem Kolben während des Arbeitszustandes zusammen. Dazu weist sie eine Kupplung auf, welche dazu geeignet ist, den Kolben im Arbeitszustand aufzunehmen. Dies trägt wiederum zum Vorteil der Platzersparnis bei, da während des Transportzustands der Kolben zum Verschließen des dem Düsen-seitigen Ende abgewandten Endes nicht mehr notwendig ist.

In einem bevorzugten Ausführungsbeispiel weist die Aufnahmehülse innenseitig eine Nut aufweist. Diese Nut kann stufenförmig, gerade oder einem konzentrischen Bogen verlaufen. Sie weist den Vorteil auf, dass der Nutzer beispielsweise bei dosierter Anwendung in der stufenförmigen Ausführung immer nur bis zum Ende eines gerade nach unten verlaufenden Teils Fluid aus der Düse gedrückt wird und bei Auftreffen des Kolbens auf eine senkrecht verlaufende Stelle das Ausbringen von Fluid gestoppt wird und der Nutzer zunächst den Kolben in Richtung der senkrecht verlaufenden Nut drehen muss. Das Drehen erfolgt solange bis der Nutzer wieder auf eine gerade nach unten verlaufende Nut stößt und die nächste Dosis des Fluids aus der Aufnahmehülse drückt. Oben bedeutet in diesem Zusammenhang, das dem Düsen-seitigen Ende abgewandte Ende und unten ist mit dem Düsen-seitigen Ende gleichzusetzen.

Der Kolben oder ein Bereich der Dichtung weist in einem bevorzugten Ausführungsbeispiel eine Kurvennocke auf, welche mit der Nut zusammenwirkt. Diese Kurvennocke dient dazu, dass der Nutzer den Kolben nur entlang der Nut bestimmungsgemäß führen kann. Vorteilhaft hierbei ist, dass auch ungeschultes Personal dosierte Fluidmengen abgeben kann.

In einem anderen bevorzugten Ausführungsbeispiel weist die Aufnahmehülse eine Griffmulde auf. Diese Griffmulde hat den Vorteil dass der Nutzer die Aufnahmehülse während des Arbeitszustandes halten kann. Da der Kolben über die Aufnahmehülse geschoben wird, fehlt der im Stand der Technik hierfür ausgebildete Ring am dem Düsen-seitigen Ende abgewandten Ende. Vorteilhaft hierbei ist, dass der Nutzer durch die Griffmulde gegen den Druck des Kolbens im Arbeitszustand sicher halten kann.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
**Figur 1** ein Ausführungsbeispiel einer Vorfüllspritze im Transportzustand.
**Figur 2** das Ausführungsbeispiel nach Figur 1 im Arbeitszustand;
**Figur 3** ein Ausführungsbeispiel einer Aufnahmehülse;
**Figur 4** ein weiteres Ausführungsbeispiel einer Vorfüllspritze;
**Figur 5** ein Ausführungsbeispiel einer erfindungsgemäßen Vorfüllspritze im Transportzustand;
**Figur 6** eine geschnittene Seitenansicht einer Aufnahmehülse aus Figur 5;
**Figur 7** eine erfindungsgemäße Vorfüllspritze im Arbeitszustand;
**Figur 8** eine geschnittene Queransicht von Figur 5;
**Figur 9** eine geschnittene Queransicht von Figur 7.

### Ausführungsbeispiele

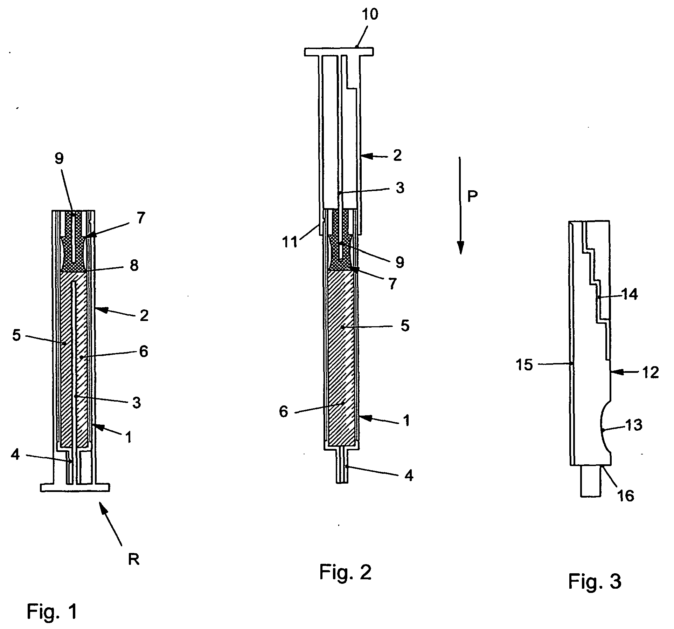

In Figur 1 ist eine Vorfüllspritze R gezeigt. Dabei handelt es sich um eine geschnittene Seitenansicht. Dort ist gut zu erkennen, wie um die Aufnahmehülse 1 ein Kolben 2 angeordnet ist. Dieser Kolben 2 ragt mit einem Teil 3 durch eine Düse 4 der Aufnahmehülse 1 in den Füllbereich 5 der Aufnahmehülse. In diesem Füllbereich 5 ist vorab ein Medium 6 eingebracht. Ausserdem ist eine Dichtung 7 zu erkennen. Diese Dichtung 7 weist auf der zum Fluid 6 weisenden Seite zumindest eine Dichtlippe 8 auf. Ausserdem ist in der Dichtung 7 eine Kupplung 9 gezeigt.

Figur 2 zeigt nun, wie der Kolben 2 von oben auf die Aufnahmehülse 1 gesetzt wird. Dabei umfasst der Kolben 2 den Umfang der Aufnahmehülse 1. Das Teil 3 ist nun in die Kupplung 9 der Dichtung 7 eingebracht. Wenn der Nutzer nun auf einen Druckpunkt 10 der Aufnahmehülse 2 drückt, wird das Teil 3 die Dichtung 7 betätigen und in Pfeilrichtung P verfahren. Dabei wird es das Medium 6, welches sich im Aufnahmeraum 5 befindet durch die Düse 4 hinausdrücken.

Ausserdem ist in Figur 2 eine Kurvennocke 11 gezeigt. In diesem Zusammenhang wird auf die Figur 3 hingewiesen. Die Figur 3 zeigt ein weiteres Ausführungsbeispiel einer erfindungsgemässen Aufnahmehülse 12. In dieser Aufnahmehülse 12 ist eine Griffmulde 13 eingeformt. Daneben ist auf der Innenseite der Aufnahmehülse 12 eine stufenförmig verlaufende Nut 14 oder eine gerade laufende Nut 15 gezeigt. Auch wenn die stufenförmige Nut 14 in Figur 3 nicht bis zum Boden 16 der Aufnahmehülse 12 verlaufend gezeigt ist, wird dies unterstellt.

Die in Figur 2 gezeigte Kurvennocke 11 dient dazu, dass der Nutzer entlang einer geraden Nut 15 oder einer stufenförmigen Nut 14 die Betätigung in Pfeilrichtung P vornehmen kann.

Dabei greift die Kurvennocke 11 in die entsprechende Nut 14 oder 15 und der Nutzer drückt auf die Aufnahmefläche 10 in Pfeilrichtung P wobei die Dichtung 7 dann entlang der entsprechenden Nut 14 oder 15 nach unten geführt wird.

Die stufenförmige Nut 14 würde hier den Vorteil aufweisen, dass ein dosiertes Ausbringen des Fluids 6 möglich wäre. Bei Eingriff der Kurvennocke 11 in die geradlinig verlaufende Nut 15 könnte der Nutzer das gesamte Fluid 6 aus der Aufnahmehülse 12 in einer Bewegung hinausdrücken. Die in Figur 3 gezeigte Aufnahmehülse 12 kann ebenso mit dem Kolben 2 betätigt werden, wie mit der Aufnahmehülse 1.

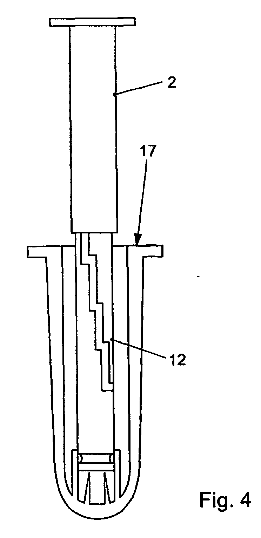

Figur 4 zeigt ein anderes Ausführungsbeispiel. Dort ist vereinfacht die Aufnahmehülse 12 und der Kolben 2 in Arbeitszustand gezeigt. Ausserdem ist zu erkennen, wie fast über den gesamten Bereich der Aufnahmehülse 12 eine Adaption 17 aufgezogen ist. Dieses Ausführungsbeispiel soll verdeutlichen, dass die erfindungsgemässe Vorrichtung auch mit herkömmlichen Adaptionen, wie sie beispielsweise bei der Vaginalanwendung zum Einsatz kommen, genutzt werden können soll.

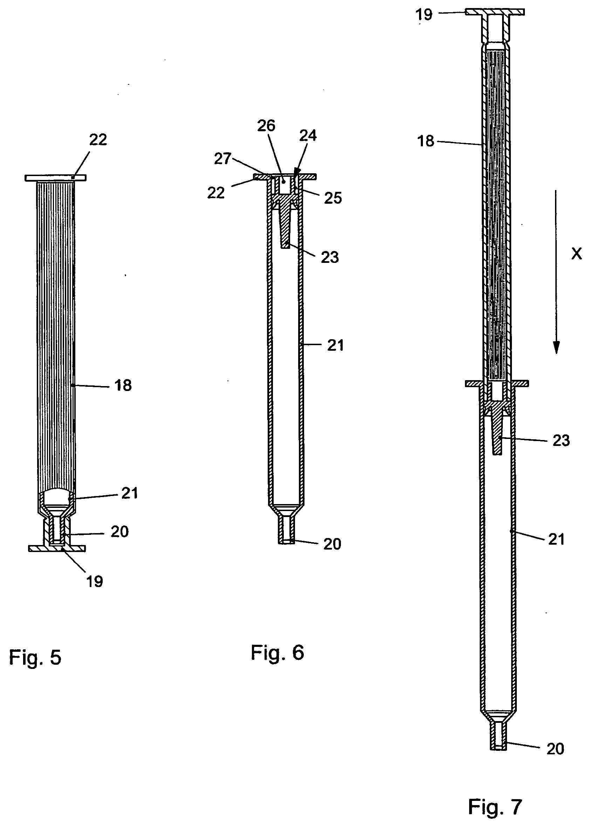

Figur 5 zeigt ein weiteres Ausführungsbeispiel einer erfindungsgemäßen Vorfüllspritze. Dort sind ein Kolben 18 sowie ein Verschluss 19 gezeigt. Der Verschluss 19 verschließt die Düse 20 der Aufnahmehülse 21, welche in Figur 5 nur teilweise geschnitten gezeigt ist. Ausserdem formt die Aufnahmehülse 21 an ihrem oberen Ende einen Ring 22 aus.

Wie in Figur 6 gut zu erkennen ist, befindet sich im Inneren der Aufnahmehülse 21 wieder eine Dichtung 23 mit einer Kupplung 24. Die Dichtung 23 verfügt außerdem über zumindest eine nicht näher beschriebene Dichtungslippe, welche das vorab eingefüllte Fluid nicht nach oben entweichen lässt. Ausserdem verfügt die Dichtung 23 über einen Kranz 25 und eine kreisförmige Ausnehmung 26. Die Innenseite der Aufnahmehülse 21 bildet mit der Außenfläche des Rings 24 einen Aufnahmeraum 27.

In Figur 7 ist nun gezeigt, wie der Kolben 18 von der Aufnahmehülse 20 gezogen wurde. Anschließend wurde die lamellenförmige Außenhaut des Kolbens 18 in der Weise verändert, dass der Außenumfang des Kolbens 18 nun entweder in den Aufnahmeraum 27 oder gar in den Ausnehmung 26 der Dichtung 23 passt.

Die Verriegelung 19 dient nun dazu, den Kolben 18 in Pfeilrichtung X zu drücken und dabei die Dichtung 23 hin zur Düse 20 zu schieben. Dabei wird ein Fluid, welches sich in der Aufnahmehülse 21 befindet, durch die Dichtung 23 aus der Düse 20 gedrückt.

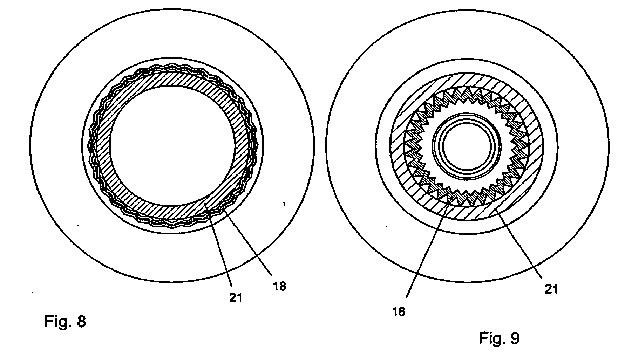

In Figur 8 ist nochmals gezeigt, wie der Kolben 18 mit einem größeren Umfang über die Aufnahmehülse 21 geschoben wurde.

Figur 9 zeigt wie der Kolben 18 mit verringertem Umfang innerhalb der Aufnahmehülse 21 gelagert ist und entlang der Innenhaut der Aufnahmehülse 21 verfahren werden kann.

**Bezugszeichenliste**

| | | | | | |
|---|---|---|---|---|---|
| 1 | Aufnahmehülse | 34 | | 67 | |
| 2 | Kolben | 35 | | 68 | |
| 3 | Teil | 36 | | 69 | |
| 4 | Düse | 37 | | 70 | |
| 5 | Füllbereich | 38 | | 71 | |
| 6 | Fluid | 39 | | 72 | |
| 7 | Dichtung | 40 | | 73 | |
| 8 | Dichtlippe | 41 | | 74 | |
| 9 | Kupplung | 42 | | 75 | |
| 10 | Druckpunkt | 43 | | 76 | |
| 11 | Kurvennocke | 44 | | 77 | |
| 12 | Aufnahmehülse | 45 | | 78 | |
| 13 | Griffmulde | 46 | | 79 | |
| 14 | Nut | 47 | | | |
| 15 | Nut | 48 | | | |
| 16 | Boden | 49 | | | |
| 17 | Adaption | 50 | | | |
| 18 | Kolben | 51 | | | |
| 19 | Verschluss | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Vorfüllspritze mit
einer Aufnahmehülse (1, 12, 21) mit einer an einem Düsen-seitigen Ende der Aufnahmehülse (1, 12, 21) angeordneten Düse (4, 20) und
einem Kolben (2, 18),
**dadurch gekennzeichnet, dass**
der Kolben (2, 18) ausgebildet ist, so dass er für einen Transportzustand an dem Düsen-seitigen Ende der Aufnahmehülse (1, 12, 21) mit der Aufnahmehülse (1, 12, 21) verbindbar ist, wobei der Umfang des Kolbens (2, 18) veränderbar ist.

2. Vorfüllspritze nach Anspruch 1, **dadurch gekennzeichnet, dass** der Kolben (2, 18) ausgebildet ist, so dass er für einen Arbeitszustand zumindest teilweise in die Aufnahmehülse (1, 12, 21) einschiebbar ist.

3. Vorfüllspritze nach einem der vorherigen Ansprüche, **dadurch gekennzeichnet, dass** in der Aufnahmehülse (1, 12, 21) eine mit dem Kolben (2, 18) für den Arbeitszustand verbindbare Dichtung (7, 23) angeordnet ist.

4. Vorfüllspritze nach Anspruch 3, **dadurch gekennzeichnet, dass** die Dichtung (7, 23) eine Kupplung (9, 24) zur Verbindung der Dichtung (7, 23) mit dem Kolben (2, 18) aufweist.

5. Vorfüllspritze nach einem der vorherigen Ansprüche, **dadurch gekennzeichnet, dass** die Aufnahmehülse (1, 12, 21) innenseitig eine Nut (14, 15) aufweist.

6. Vorfüllspritze nach Anspruch 5, **dadurch gekennzeichnet, dass** die Nut (14, 15) stufenförmig abgesetzt oder gerade verläuft.

7. Vorfüllspritze nach Anspruch 5 oder 6, **dadurch gekennzeichnet, dass** der Kolben (2, 18) eine Kurvennocke aufweist, die ausgebildet ist, um mit der Nut (14, 15) zusammenzuwirken.

8. Vorfüllspritze nach einem der vorherigen Ansprüche, **dadurch gekennzeichnet, dass** die Aufnahmehülse (1, 12, 21) eine Griffmulde (13) aufweist.

9. Verwendung einer Vorfüllspritze nach einem der Ansprüche 1 bis 8, **gekennzeichnet durch** folgende Schritte:
- Abziehen des Kolbens (2, 18) von der Aussenseite der Aufnahmehülse (1, 12, 21),
- Einschieben zumindest eines Teil des Kolbens (2, 18) in die Aufnahmehülse (1,12, 21),
- Ausbringen eines in der Aufnahmehülse (1, 12, 21) befindlichen Fluids (6) **durch** Betätigen des Kolbens (2, 18).
- vor dem Schritt des Einschiebens und nach dem Schritt des Abziehens wird ein Umfang des Kolbens (2, 18) verändert.

## Claims

1. Prefill syringe comprising
a receiving tube (1, 12, 21) having a nozzle (4, 20) positioned at the nozzle-side end of the receiving tube (1, 12, 21) and
a plunger (2, 18)
**characterised in that**
the plunger (2, 18) is formed in such a way that it can be connected to the receiving tube (1, 12, 21) at the nozzle-side end of the receiving tube (1, 12, 21) for a transport state, the size of the plunger (2, 18) being variable.

2. Prefill syringe according to claim 1, **characterised in that** the plunger (2, 18) is formed in such a way that it can be inserted into the receiving tube (1, 12, 21) at least in part for an operating state.

3. Prefill syringe according to either of the preceding claims, **characterised in that** a seal (7, 23) which is connectable to the plunger (2, 18) for the operating state is arranged in the receiving tube (1, 12, 21).

4. Prefill syringe according to claim 3, **characterised in that** the seal (7, 23) comprises a coupling (9, 24) for connecting the seal (7, 23) to the plunger (2, 18).

5. Prefill syringe according to any of the preceding claims, **characterised in that** the receiving tube (1, 12, 21) has a groove (14, 15) on the inside.

6. Prefill syringe according to claim 5, **characterised in that** the groove (14, 15) extends offset in a stepped shape or straight.

7. Prefill syringe according to either claim 5 or claim 6, **characterised in that** the plunger (2, 18) comprises a curved cam which is formed to cooperate with the groove (14, 15).

8. Prefill syringe according to any of the preceding claims, **characterised in that** the receiving tube (1, 12, 21) comprises a recessed grip (13).

9. Use of a prefill syringe according to any of claims 1 to 8, **characterised by** the following steps:
- withdrawing the plunger (2, 18) from the outside of the receiving tube (1, 12, 21),
- inserting at least part of the plunger (2, 18) into the receiving tube (1, 12, 21),
- extracting a fluid (6) located in the receiving tube (1, 12, 21) by actuating the piston (2, 18).
a size of the plunger (2, 18) is changed before the insertion step and after the withdrawal step.

## Revendications

1. Seringue de pré-remplissage comportant :
un manchon de réception (1, 12, 21) comprenant une buse (4, 20) disposée au niveau d'une extrémité du manchon de réception (1, 12, 21) située côté buse, et
un piston (2, 18),
**caractérisée en ce que**
le piston (2, 18) est réalisé de telle sorte qu'il peut être relié au manchon de réception (1, 12, 21) au niveau de l'extrémité du manchon de réception (1, 12, 21) qui est située côté buse, pour un état de transport, la circonférence du piston (2, 18) pouvant être modifiée.

2. Seringue de pré-remplissage selon la revendication 1, **caractérisée en ce que** le piston (2, 18) est réalisé de telle sorte qu'il peut être inséré au moins partiellement dans le manchon de réception (1, 12, 21) pour un état de travail.

3. Seringue de pré-remplissage selon une des revendications précédentes, **caractérisée en ce que**, dans le manchon de réception (1, 12, 21), est disposé un joint d'étanchéité (7, 23) qui peut être relié au piston (2, 18) pour l'état de travail.

4. Seringue de pré-remplissage selon la revendication 3, **caractérisée en ce que** le joint d'étanchéité (7, 23) présente un accouplement (9, 24) destiné à relier le joint d'étanchéité (7, 23) au piston (2, 18).

5. Seringue de pré-remplissage selon une des revendications précédentes, **caractérisée en ce que** le manchon de réception (1, 12, 21) présente à l'intérieur une rainure (14, 15).

6. Seringue de pré-remplissage selon la revendication 5, **caractérisée en ce que** la rainure (14, 15) s'étend de manière étagée ou droite.

7. Seringue de pré-remplissage selon la revendication 5 ou 6, **caractérisée en ce que** le piston (2, 18) présente une came incurvée réalisée pour coopérer avec la rainure (14, 15).

8. Seringue de pré-remplissage selon une des revendications précédentes, **caractérisée en ce que** le manchon de réception (1, 12, 21) présente une poignée creuse (13).

9. Utilisation d'une seringue de pré-remplissage selon une des revendications 1 à 8, **caractérisée par** les étapes suivantes :
- retrait du piston (2, 18) de la face extérieure du manchon de réception (1, 12, 21),
- insertion d'au moins une partie du piston (2, 18) dans le manchon de réception (1, 12, 21),
- extraction d'un fluide (6) se trouvant dans le manchon de réception (1, 12, 21) en actionnant le piston (2, 18),
- avant l'étape d'insertion et après l'étape de retrait, une circonférence du piston (2, 18) est modifiée.
